# EUROPEAN PATENT SPECIFICATION

(11) **EP 0 295 117 B1**
(45) Date of publication and mention of the grant of the patent: **05.04.2000**
(21) Application number: 88305306.8
(22) Date of filing: 10.06.1988
(51) Int. Cl.: C07D 231/44, A01N 43/56

(54) **Derivative of a N-phenylpyrazole**
N-Phenylpyrazol-Derivat
Dérivé de N-phénylpyrazole

(30) Priority: 12.06.1987 GB 8713768
(43) Date of publication of application: 14.12.1988
(60) Divisional application: 99113797.7
(73) Proprietor: RHONE-POULENC AGRICULTURE LIMITED, Ongar, Essex CM5 0HW (GB)
(72) Inventor: Buntain, Ian George, Dagenham Essex RM10 7XS (GB); Hatton, Leslie Roy, Dagenham Essex RM10 7XS (GB); Hawkins, David William, Dagenham Essex RM10 7XS (GB); Pearson, Christopher John, Dagenham Essex RM10 7XS (GB); Roberts, David Alan, Dagenham Essex RM10 7XS (GB)
(74) Representative: Bentham, Stephen

(56) References cited:
- EP-A- 0 201 852
- EP-A- 0 234 119
- EP-A- 0 245 785

## Description

This invention relates to an N-phenylpyrazole derivative, to compositions containing it and to its use against arthropod, plant nematode, helminth and protozoan pests.

EP-A-201 852 (Bayer) discloses, as insecticides, acaricides and nematocides, 1-aryl-5-aminopyrazoles, in which the 3-position is unsubstituted or substituted by an alkyl or haloalkyl group and the 4-position is substituted by alkyl, aralkyl or aryl sulphenyl, sulphinyl or sulphonyl.

EP-A-245 875 (also Bayer) discloses, as acaricides, 1-aryl-4-cyano-5-aminopyrazoles in which the 3-position is substituted by alkyl sulphenyl, sulphinyl or sulphonyl.

The applicant's earlier patent application EP-A-234,119 which was published after the priority date of the present application discloses a method for the control of arthropod, plant nematode or helminth pests using compounds of formula: 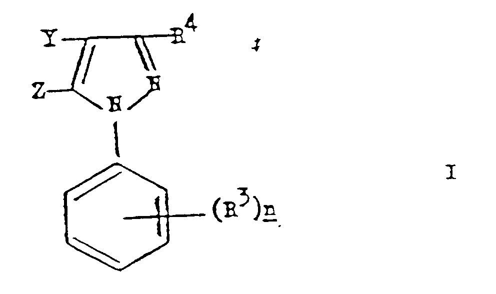 wherein Y is halogen, cyano, nitro, RSO_{2,} RSO or RS in which R is alkyl, cycloalkyl or alkenyl, thiocyanato, sulphamoyl, carbamoyl, alkoxycarbonyl, alkanoyl or alkyl; Z represents hydrogen, an amino group -NR¹R², alkylsulphenylamino, alkoxymethyleneamino, halogen, alkyl, carboxyl, alkylthio, alkylsulphinyl or alkylsulphonyl, trialkylsilymethyl, trialkylsilyl, cyano or nitro; R³ represents halogen, alkyl or alkoxy, halogen-substituted alkylthio or alkylsulphinyl, nitro, cyano or alkysulphonyl; R⁴ represents halogen, cyano, nitro, alkyl or cycloalkyl; and n is 1 to 5, and salts thereof, provided that R⁴, Y and Z do not simultaneously represent one of nitro, cyano, halogen and unsubstituted alkyl.

The present invention provides an N-phenylpyrazole derivative of formula (I) 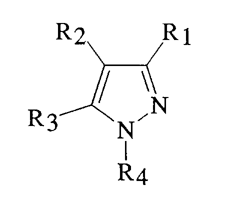 in which R¹ represents a cyano group; R² represents a group R⁵SO in which R⁵ represents a trifluoromethyl group; R³ represents an amino group; and R⁴ represents a 2,6-dichloro-4-trifluoromethylphenyl group, i.e. the compound 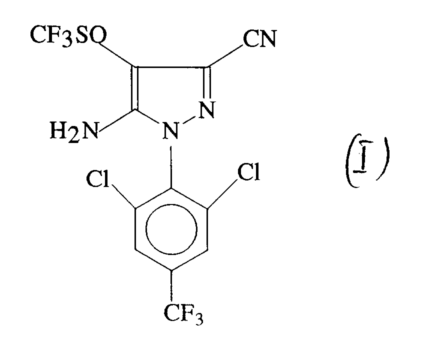 This compound has valuable activity against arthropod, plant nematode, helminth and protozoan pests, more particularly by ingestion of the compound of formula (I) by arthropods.

The compound of formula (I), processes for its preparation, compositions containing it and methods for its use constitute features of the present invention.

According to a feature of the present invention, there is provided a method for the control of arthropod, plant nematode, helminth or protozoan pests at a locus which comprises the treatment of the locus (e.g. by application or administration) with an effective amount of the compound of formula (I), with the exclusion of a method for the treatment of the human or animal body carried out by a medical or veterinary practitioner as therapy. The compound of formula (I) may, in particular, be used in the field of veterinary medicine and livestock husbandry and in the maintenance of public health against arthropods, helminths or protozoa which are parasitic internally or externally upon vertebrates, particularly warm-blooded vertebrates, for example man and domestic animals, e.g. cattle, sheep, goats, equines, swine, poultry, dogs, cats and fishes, for example Acarina, including ticks (e.g. Ixodes spp., Boophilus spp. e.g. Boophilus microplus, Amblyomma spp., Hyalomma spp., Rhipicephalus spp. e.g. Rhipicephalus appendiculatus, Haemaphysalis spp., Dermacentor spp., Ornithodorus spp. (e.g. Ornithodorus moubata and mites (e.g. Damalinia spp., Dermahyssus gallinae, Sarcoptes spp. e.g. Sarcoptes scabiei, Psoroptes spp., Chorioptes spp., Demodex spp., Eutrombicula spp.,); Diptera (e.g. Aedes spp., Anopheles spp., Musca spp., Hypoderma spp., Gasterophilus spp., Simulium spp.); Hemiptera (e.g. Triatoma spp.); Phthiraptera (e.g. Damalinia spp., Linognathus spp.); Siphonaptera (e.g. Ctenocephalides spp.): Dictyoptera (e.g. Periplaneta spp., Blatella spp.); Hymenoptera (e.g. Monomorium pharaonis); for example against infections of the gastro-intestinal tract caused by parasitic nematode worms, for example members of the family Trichostrongylidae, Nippostrongylus brasiliensis, Trichinella spiralis, Haemonchus contortus, Trichostrongylus colubriformis, Nematodirus battus, Ostertagia circumcincta, Trichostroncylus axei, Cooperia spp. and Hymenolepis nana; in the control and treatment of protozoal diseases caused by, for example, Eimeria spp. e.g. Eimeria tenella, Eimeria acervulina, Eimeria brunetti, Eimeria maxima and Eimeria necatrix, Trypanosoma cruzi, Leishmania spp., Plasmodium spp., Babesia spp., Trichomonadidae spp., Histomonas spp., Giardia spp., Toxoplasma spp., Entamoeba histolytica and Theileria spp.; in the protection of stored products, for example cereals, including grain and flour, groundnuts, animal feedstuffs, timber and household goods, e.g. carpets and textiles, against attack by arthropods, more especially beetles, including weevils, moths and mites, for example Ephestia spp. (flour moths), Anthrenus spp. (carpet beetles), Tribolium spp. (flour beetles), Sitophilus spp. (grain weevils) and Acarus spp. (mites), in the control of cockroaches, ants and termites and similar arthropod pests in infested domestic and industrial premises and in the control of mosquito larvae in waterways, wells, reservoirs or other running or standing water; for the treatment of foundations, structure and soil in the prevention of the attack on buildings by termites, for example, Reticulitermes spp., Heterotermes spp., Coptotermes spp.; in agriculture, against adults, larvae and eggs of Lepidoptera (butterflies and moths), e.g. Heliothis spp. such as Heliothis virescens (tobacco budworm), Heliothis armigera and Heliothis zea, Spodoptera spp. such as S.exempta, S.littoralis (Egyptian cotton worm), S.eridania (southern army worm), Mamestra configurata (bertha army worm); Earias spp. e.g. E.insulana (Egyptian bollworm), Pectinophora spp. e.g. Pectinophora gossypiella (pink bollworm), Ostrinia spp. such as O.nubilalis (European cornborer), Trichoplusiani (cabbage looper), Pieris spp. (cabbage worms), Laphygma spp. (army worms), Agrotis and Amathes spp. (cutworms), Wiseana spp. (porina moth), Chilo spp. (rice stem borer), Tryporyza spp. and Diatraea spp. (sugar cane borers and rice borers), Sparganothis pilleriana (grape berry moth), Cydia pomonella (codling moth), Archips spp. (fruit tree tortrix moths), Plutella xylostella (diamond back moth); against adult and larvae of Coleoptera (beetles) e.g. Hypothenemus hampei (coffee berry borer), Hylesinus spp. (bark beetles), Anthonomus grandis (cotton boll weevil), Acalymma spp. (cucumber beetles), Lema spp., Psylliodes spp., Leptinotarsa decemlineata (Colorado potato beetle), Diabrotica spp. (corn rootworms), Gonocephalum spp. (false wire worms), Agriotes spp. (wireworms), Dermolepida and Heteronychus spp. (white grubs), Phaedon cochleariae (mustard beetle), Lissorhoptrus oryzophilus (rice water weevil), Meligethes spp. (pollen beetles), Ceutorhynchus spp., Rhynchophorus and Cosmopolites spp. (root weevils); against Hemiptera e.g. Psylla spp., Bemisia spp., Trialeurodes spp., Aphis spp., Myzus spp., Megoura viciae, Phylloxera spp., Adelges spp., Phorodon humuli (hop damson aphid), Aeneolamia spp., Nephotettix spp. (rice leaf hoppers), Empoasca spp., Nilaparvata spp., Perkinsiella spp., Pyrilla spp., Aonidiella spp. (red scales), Coccus spp., Pseucoccus spp., Helopeltis spp. (mosquito bugs), Lygus spp., Dysdercus spp., Oxycarenus spp., Nezara spp.; Hymenoptera e.g. Athalia spp. and Cephus spp. (saw flies), Atta spp. (leaf cutting ants); Diptera e.g. Hylemyia spp. (root flies), Atherigona spp. and Chlorops spp. (shoot flies), Phytomyza spp. (leaf miners), Ceratitis spp. (fruit flies); Thysanoptera such as Thrips tabaci; Orthoptera such as Locusta and Schistocerca spp. (locusts) and crickets e.g. Gryllus spp. and Acheta spp.; Collembola e.g. Sminthurus spp. and Onychiurus spp. (springtails), Isoptera e.g. Odontotermes spp. (termites), Dermaptera e.g. Forficula spp. (earwigs) and also other arthropods of agricultural significance such as Acari (mites) e.g. Tetranychus spp., Panonychus spp. and Bryobia spp. (spider mites), Eriophyes spp. (gall mites), Polyphagotarsonemus spp.; Blaniulus spp. (millipedes), Scutigerella spp. (symphilids), Oniscus spp. (woodlice) and Triops spp. (crustacea); nematodes which attack plants and trees of importance to agriculture, forestry and horticulture either directly or by spreading bacterial, viral, mycoplasma or fungal diseases of the plants, root-knot nematodes such as Meloidogyne spp. (e.g. M. incognita); cyst nematodes such as Globodera spp. (e.g. G. rostochiensis); Heterodera spp. (e.g. H. avenae); Radopholus spp. (e.g. R. similis); lesion nematodes such as Pratylenchus spp. (e.g. P. pratensis); Belonolaimus spp. (e.g. B. gracilis); Tylenchulus spp. (e.g. T. semipenetrans); Rotylenchulus spp. (e.g. R. reniformis); Rotylenchus spp. (e.g. R. robustus); Helicotylenchus spp. (e.g. H. multicinctus); Hemicycliophora spp. (e.g. H. gracilis); Criconemoides spp. (e.g. C. similis); Trichodorus spp. (e.g. T. primitivus); dagger nematodes such as Xiphinema spp. (e.g. X. diversicaudatum), Longidorus spp. (e.g. L. elongatus); Hoplolaimus spp. (e.g. H. coronatus); Aphelenchoides spp. (e.g. A. ritzema-bosi, A. besseyi); stem and bulb eelworms such as Ditylenchus spp. (e.g. D. dipsaci).

The invention also provides a method for the control of arthropod or nematode pests of plants which comprises the application to the plants or to the medium in which they grow of an effective amount of the compound of formula (I).

For the control of arthropods and nematodes, the active compound is generally applied to the locus in which arthropod or nematode infestation is to be controlled at a rate of 0.1kg to 25kg of active compound per hectare of locus treated. Under ideal conditions, depending on the pest to be controlled, the lower rate may offer adequate protection. On the other hand, adverse weather conditions, resistance of the pest and other factors may require that the active ingredient be used in higher proportions. In foliar application, a rate of 1g to 1000g/ha may be used.

When the pest is soil-borne, the formulation containing the active compound is distributed evenly over the area to be treated in any convenient manner. Application may be made, if desired, to the field or crop-growing area generally or in close proximity to the seed or plant to be protected from attack. The active component can be washed into the soil by spraying with water over the area or can be left to the natural action of rainfall. During or after application, the formulation can, if desired, be distributed mechanically in the soil, for example by ploughing or disking. Application can be prior to planting, at planting, after planting but before sprouting has taken place or after sprouting.

The compound of formula (I) may be applied in solid or liquid compositions to the soil principally to control those nematodes dwelling therein but also to the foliage principally to control those nematodes attacking the aerial parts of the plants (e.g. Aphelenchoides spp. and Ditylenchus spp. listed above).

The compound of formula (I) is of value in controlling pests which feed on parts of the plant remote from the point of application, e.g. leaf feeding insects are killed by the subject compounds applied to roots.

In addition the compound may reduce attacks on the plant by means of antifeeding or repellent effects.

The compound of formula (I) is of particular value in the protection of field, forage, plantation, glasshouse, orchard and vineyard crops, of ornamentals and of plantation and forest trees, for example, cereals (such as maize, wheat, rice, sorghum), cotton, tobacco, vegetables and salads (such as beans, cole crops, curcurbits, lettuce, onions, tomatoes and peppers), field crops (such as potato, sugar beet, ground nuts, soyabean, oil seed rape), sugar cane, grassland and forage (such as maize, sorghum, lucerne), plantations (such as of tea, coffee, cocoa, banana, oil palm, coconut, rubber, spices), orchards and groves (such as of stone and pip fruit, citrus, kiwifruit, avocado, mango, olives and walnuts), vineyards, ornamental plants, flowers and shrubs under glass and in gardens and parks, forest trees (both deciduous and evergreen) in forests, plantations and nurseries.

It is also valuable in the protection of timber (standing, felled, converted, stored or structural) from attack by sawflies (e.g. Urocerus) or beetles (e.g. scolytids, platypodids, lyctids, bostrychids, cerambycids, anobiids), or termites, for example, Reticulitermes spp., Heterotermes spp., Coptotermes spp.

It has applications in the protection of stored products such as grains, fruits, nuts, spices and tobacco, whether whole, milled or compounded into products, from moth, beetle and mite attack. Also protected are stored animal products such as skins, hair, wool and feathers in natural or converted form (e.g. as carpets or textiles) from moth and beetle attack; also stored meat and fish from beetle, mite and fly attack.

The compound of general formula (I) is of particular value in the control of arthropods, helminths or protozoa which are injurious to, or spread or act as vectors of diseases in man and domestic animals, for example those hereinbefore mentioned, and more especially in the control of ticks, mites, lice, fleas, midges and biting, nuisance and myiasis flies. The compound of general formula (I) is particularly useful in controlling arthropods, helminths or protozoa which are present inside domestic host animals or which feed in or on the skin or suck the blood of the animal, for which purpose they may be administered orally, parenterally, percutaneously or topically.

Coccidiosis, a disease caused by infections by protozoan parasites of the genus Eimeria, is an important potential cause of economic loss in domestic animals and birds, particularly those raised or kept under intensive conditions. For example, cattle, sheep, pigs and rabbits may be affected, but the disease is especially important in poultry, in particular chickens.

The poultry disease is generally spread by the birds picking up the infectious organism in droppings on contaminated litter or ground or by way of food or drinking water. The disease is manifested by hemorrhage, accumulation of blood in the ceca, passage of blood to the droppings, weakness and digestive disturbances. The disease often terminates in the death of the animal but the fowl which survive severe infections have had their market value substantially reduced as a result of the infection.

Administration of a small amount of the compound of general formula (I) preferably by combination with poultry feed is effective in preventing or greatly reducing the incidence of coccidiosis. The compound is effective against both the cecal form (caused by E. tenella) and the intestinal forms (principally caused by E. acervulina, E. brunetti, E. maxima and E. necatrix).

The compound of general formula (I) also exerts an inhibitory effect on the oocysts by greatly reducing the number and or the sporulation of those produced.

The compositions hereinafter described for topical application to man and animals and in the protection of stored products, household goods, property and areas of the general environment may, in general, alternatively be employed for application to growing crops and crop growing loci and as a seed dressing.

Suitable means of applying the compound of formula (I) include:-
to persons or animals infested by or exposed to infestation by arthropods, helminths or protozoa, by parenteral, oral or topical application of compositions in which the active ingredient exhibits an immediate and/or prolonged action over a period of time against the arthropods, helminths or protozoa, for example by incorporation in feed or suitable orally-ingestible pharmaceutical formulations, edible baits, salt licks, dietary supplements, pour-on formulations, sprays, baths, dips, showers, jets, dusts, greases, shampoos, creams, wax-smears and livestock self-treatment systems;
to the environment in general or to specific locations where pests may lurk, including stored products, timber, household goods, and domestic and industrial premises, as sprays, fogs, dusts, smokes, wax-smears, lacquers, granules and baits, and in tricklefeeds to waterways, wells, reservoirs and other running or standing water;
to domestic animals in feed to control fly larvae feeding in their faeces;
to growing crops as foliar sprays, dusts, granules, fogs and foams; also as suspensions of finely divided and encapsulated compound of formula (I); as soil and root treatments by liquid drenches, dusts, granules, smokes and foams; and as seed dressings by liquid slurries and dusts.

The compound of formula (I) may be applied to control arthropods, helminths or protozoa in compositions of any type known to the art suitable for internal or external administration to vertebrates or application for the control of arthropods in any premises or indoor or outdoor area, containing as active ingredient the compound of general formula (I) in association with one or more compatible diluents or adjuvants appropriate for the intended use. All such compositions may be prepared in any manner known to the art.

Compositions suitable for administration to vertebrates or man include preparations suitable for oral, parenteral, percutaneous, e.g. pour-on, or topical administration.

Compositions for oral administration comprise the compound of formula (I) in association with pharmaceutically acceptable carriers or coatings and include, for example, tablets, pills, capsules, pastes, gels, drenches, medicated feeds, medicated drinking water, medicated dietary supplements, slow-release boluses or other slow-release devices intended to be retained within the gastro-intestinal tract. Any of these may incorporate active ingredient contained within microcapsules or coated with acid-labile or alkali-labile or other pharmaceutically acceptable enteric coatings. Feed premixes and concentrates containing compounds of the present invention for use in preparation of medicated diets, drinking water or other materials for consumption by animals may also be used.

Compositions for parenteral administration include solutions, emulsions or suspensions in any suitable pharmaceutically acceptable vehicle and solid or semisolid subcutaneous implants or pellets designed to release active ingredient over a protracted period and may be prepared and made sterile in any appropriate manner known to the art.

Compositions for percutaneous and topical administration include sprays, dusts, baths, dips, showers, jets, greases, shampoos, creams, wax-smears, or pour-on preparations and devices (e.g. ear tags) attached externally to animals in such a way as to provide local or systemic arthropod control.

Solid or liquid baits suitable for controlling arthropods comprise the compound of formula (I) and a carrier or diluent which may include a food substance or some other substance to induce comsumption by the arthropod.

Liquid compositions include water miscible concentrates, emulsifiable concentrates, flowable suspensions, wettable or soluble powders containing the compound of formula (I) which may be used to treat substrates or sites infested or liable to infestation by arthropods including premises, outdoor or indoor storage or processing areas, containers or equipment and standing or running water.

Solid homogenous or heterogenous compositions containing the compound of formula (I), for example granules, pellets, briquettes or capsules, may be used to treat standing or running water over a period of time. A similar effect may be achieved using trickle or intermittent feeds of water dispersible concentrates as described herein.

Compositions in the form of aerosols and aqueous or non-aqueous solutions or dispersions suitable for spraying, fogging and low- or ultra-low volume spraying may also be used.

Suitable solid diluents which may be used in the preparation of compositions suitable for applying the compound of formula (I) include aluminium silicate, kieselguhr, corn husks, tricalcium phosphate, powdered cork, absorbent carbon black, magnesium silicate, a clay such as kaolin, bentonite or attapulgite, and water soluble polymers and such solid compositions may, if desired, contain one or more compatible wetting, dispersing, emulsifying or colouring agents which, when solid, may also serve as diluent.

Such solid compositions, which may take the form of dusts, granules or wettable powders, are generally prepared by impregnating the solid diluents with solutions of the compound of formula (I) in volatile solvents, evaporating the solvents and, if necessary, grinding the products so as to obtain powders and, if desired, granulating or compacting the products so as to obtain granules, pellets or briquettes or by encapsulating finely divided active ingredient in natural or synthetic polymers, e.g. gelatin, synthetic resins and polyamides.

The wetting, dispersing and emulsifying agents which may be present, particularly in wettable powders, may be of the ionic or non-ionic types, for example sulphoricinoleates, quaternary ammonium derivatives or products based upon condensates of ethylene oxide with nonyl- and octyl-phenol, or carboxylic acid esters of anhydrosorbitols which have been rendered soluble by etherification of the free hydroxy groups by condensation with ethylene oxide, or mixtures of these types of agents. Wettable powders may be treated with water immediately before use to give suspensions ready for application.

Liquid compositions for the application of the compound of formula (I) may take the form of solutions, suspensions and emulsions of the compound of formula (I) optionally encapsulated in natural or synthetic polymers, and may, if desired, incorporate wetting, dispersing or emulsifying agents. These emulsions, suspensions and solutions may be prepared using aqueous, organic or aqueous-organic diluents, for example acetophenone, isophorone, toluene, xylene, mineral, animal or vegetable oils, and water soluble polymers (and mixtures of these diluents), which may contain wetting, dispersing or emulsifying agents of the ionic or non-ionic types or mixtures thereof, for example those of the types described above. When desired, the emulsions containing the compound of formula (I) may be used in the form of self-emulsifying concentrates containing the active substance dissolved in the emulsifying agents or in solvents containing emulsifying agents compatible with the active substance, the simple addition of water to such concentrates producing compositions ready for use.

Compositions containing the compound of formula (I) which may be applied to control arthropod, plant nematode, helminth or protozoan pests, may also contain synergists (e.g. piperonyl butoxide or sesamex), stabilizing substances, other insecticides, acaricides, plant nematocides, anthelmintics or anticoccidials, fungicides (agricultural or veterinary as apropriate e.g. benomyl, iprodione), bactericides, arthropod or vertebrate attractants or repellents or pheromones, reodorants, flavouring agents, dyes and auxiliary therapeutic agents, e.g. trace elements. These may be designed to improve potency, persistence, safety, uptake where desired, spectrum of pests controlled or to enable the composition to perform other useful functions in the same animal or area treated.

Examples of other pesticidally-active compounds which may be included in, or used in conjunction with, the compositions of the present invention are:-acephate, chlorpyrifos, demeton-S-methyl, disulfoton, ethoprofos, fenitrothion, malathion, monocrotophos, parathion, phosalone, pirimiphos-methyl, triazophos, cyfluthrin, cypermethrin, deltamethrin, fenpropathrin, fenvalerate, permethrin, aldicarb, carbosulfan, methomyl, oxamyl, pirimicarb, bendiocarb, teflubenzuron, dicofol, endosulfan, lindane, benzoximate, cartap, cyhexatin, tetradifon, avermectins, ivermectin, milbemycins, thiophanate, trichlorfon, dichlorvos, diaveridine and dimetridazole.

The compositions for application to control arthropod, plant nematode, helminth or protozoan pests usually contain from 0.00001% to 95%, more particularly from 0.0005% to 50%, by weight of the compound of formula (I) together with other substances toxic to arthropods and plant nematodes, anthelmintics, anticoccidials, synergists, trace elements or stabilisers). The actual compositions employed and their rate of application will be selected to achieve the desired effect(s) by the farmer, livestock producer, medical or veterinary practitioner, pest control operator or other person skilled in the art. Solid and liquid compositions for application topically to animals, timber, stored products or household goods usually contain from 0.00005% to 90%, more particularly from 0.001% to 10%, by weight of the compound of formula (I). For administration to animals orally or parenterally, including percutaneously solid and liquid compositions normally contain from 0.1% to 90% by weight of the compound of formula (I). Medicated feedstuffs normally contain from 0.001% to 3% by weight of the compound of formula (I). Concentrates and supplements for mixing with feedstuffs normally contain from 5% to 90%, and preferably from 5% to 50%, by weight of the compound of formula (I). Mineral salt licks normally contain from 0.1% to 10% by weight of the compound of formula (I).

Dusts and liquid compositions for application to livestock, persons, goods, premises or outdoor areas may contain 0.0001% to 15%, and more especially 0.005% to 2.0%, by weight of the compound of formula (I). Suitable concentrations in treated waters are between 0.0001 ppm and 20 ppm, and more especially 0.001 ppm to 5.0 ppm of the compound of formula (I) and may also be used therapeutically in fish farming with appropriate exposure times. Edible baits may contain from 0.01% to 5% and preferably 0.01% to 1.0%, by weight of the compound of formula (I).

When administered to vertebrates parenterally or orally or by percutaneous or other means, the dosage of the compound of general formula (I) will depend upon the species, age and health of the vertebrate and upon the nature and degree of its actual or potential infestation by arthropod, helminth or protozoan pest. A single dose of 0.1 to 100 mg, preferably 2.0 to 20.0 mg, per kg body weight of the animal or doses of 0.01 to 20.0 mg, preferably 0.1 to 5.0 mg, per kg body weight of the animal per day for sustained medication are generally suitable by oral or parenteral administration. By use of sustained release formulations or devices, the daily doses required over a period of months may be combined and administered to animals on a single occasion.

In experiments on activity against arthropods carried out on the compound of formula (I), the following results (wherein ppm indicates the concentration of the compound in parts per million of the test solution applied) have been obtained:-

### Test

One or more dilutions of the compound of formula (I) were made in 50% aqueous acetone.

### (a) Test species: Plutella xylostella (Diamond-back Moth).

Turnip leaf discs were set in agar in petri-dishes and infected with 10 larvae (2nd instar Plutella). Four replicate dishes were assigned to each treatment and were sprayed under a Potter Tower with the appropriate test dilution. Four or five days after treatment the dishes were removed from constant temperature (25°C) room in which they had been held and the mean percentage mortalities of larvae were determined. These data were corrected against the mortalities in dishes treated with 50% aqueous acetone alone which served as controls.

According to the above method an application of the compound of formula (I) was effective against the larvae of Plutella xylostella producing at least 65% mortality at less than 500ppm.

The following composition Examples illustrate compositions for use against arthropod, plant nematode, helminth or protozoan pests which comprise, as active ingredient, the compound of general formula (I). The compositions described in Composition Examples 1 to 6 can each be diluted in water to give a sprayable composition at concentrations suitable for use in the field.

### COMPOSITION EXAMPLE 1

A water soluble concentrate was prepared from

| | |
|---|---|
| the compound of general formula (I) | 7% w/v |
| Ethylan BCP | 10% w/v |
| and N-methylpyrrolidone | to 100% by volume |

by dissolving the Ethylan BCP in a portion of N-methylpyrrolidone, and then adding the active ingredient with heating and stirring until dissolved. The resulting solution was made up to volume by adding the remainder of the solvent.

### COMPARISON EXAMPLE 2

An emulsifiable concentrate was prepared from

| | |
|---|---|
| the compound of general formula (I) | 7% w/v |
| Soprophor BSU | 4% w/v |
| Arylan CA | 4% w/v |
| N-methylpyrrolidone | 50% w/v |

and Solvesso 150 to 100% by volume by dissolving Soprophor BSU, Arylan CA and the active ingredient in N-methylpyrrolidone, and then adding Solvesso 150 to volume.

### COMPOSITION EXAMPLE 3

A wettable powder was prepared from

| | |
|---|---|
| the compound of formula (I) | 40% w/w |
| Arylan S | 2% w/w |
| Darvan No. 2 | 5% w/w |
| and Celite PF | to 100% by weight |

by mixing the ingredients, and grinding the mixture in a hammer-mill to a particle size less than 50 microns.

### COMPOSITION EXAMPLE 4

An aqueous flowable formulation was prepared from

| | |
|---|---|
| the compound of formula (I) | 30% w/v |
| Ethylan BCP | 1% w/v |
| Sopropon T36 | 0.2% w/v |
| Ethylene glycol | 5% w/v |
| Rhodigel 23 | 0.15% w/v |
| and Water | to 100% by volume |

by intimately mixing the ingredients and grinding in a bead mill until the median particle size was less than 3 microns.

### COMPOSITION EXAMPLE 5

An emulsifiable suspension concentrate was prepared from

| | |
|---|---|
| the compound of formula (I) | 30% w/v |
| Ethylan BCP | 10% w/v |
| Bentone 38 | 0.5% w/v |
| and Solvesso 150 | to 100% by volume |

by intimately mixing the ingredients and grinding in a bead mill until the median particle size was less than 3 microns.

### COMPOSITION EXAMPLE 6

Water dispersible granules were prepared from

| | |
|---|---|
| the compound of formula (I) | 30% w/w |
| Darvan No. 2 | 15% w/w |
| Arylan S | 8% w/w |
| and Celite PF | to 100% by weight |

by mixing the ingredients, micronising in a fluid-energy mill, and then granulating in a rotating pelletiser by spraying on sufficient water (up to 10% w/w). The resulting granules were dried in a fluid-bed drier to remove excess water.

Descriptions of commercial ingredients used in the foregoing Composition Examples:-
- Ethylan BCP: nonylphenol ethylene oxide condensate
- Soprophor BSU: condensate of tristyrylphenol and ethylene oxide
- Arylan CA: 70% w/v solution of calcium dodecylbenzenesulphonate
- Solvesso 150: light C₁₀-aromatic solvent
- Arylan S: sodium dodecylbenzenesulphonate
- Darvan: sodium lignosulphonate
- Celite PF: synthetic magnesium silicate carrier
- Sopropon T36: sodium salt of polycarboxylic acid
- Rhodigel 23: polysaccharide xanthan gum
- Bentone 38: organic derivative of magnesium montmorillonite

### COMPOSITION EXAMPLE 7

A dusting powder may be prepared by intimately mixing:-

| | |
|---|---|
| the compound of formula (I) | 1 to 10% w/w (weight/weight) |
| Talc superfine | to 100% by weight |

This powder may be applied to a locus of arthropod infestation, for example refuse tips or dumps, stored products or household goods or animals infested by, or at risk of infestation by, arthropods to control the arthropods by oral ingestion. Suitable means for distributing the dusting powder to the locus of arthropod infestation include mechanical blowers, handshakers and livestock self treatment devices.

### COMPOSITION EXAMPLE 8

An edible bait may be prepared by intimately mixing:-

| | |
|---|---|
| the compound of formula (I) | 0.1 to 1.0% w/w |
| Wheat flour | 80% w/w |
| Molasses | to 100% w/w |

This edible bait may be distributed at a locus, for example domestic and industrial premises, e.g. kitchens, hospitals or stores, or outdoor areas, infested by arthropods, for example ants, locusts, cockroaches and flies, to control the arthropods by oral ingestion.

### COMPOSITION EXAMPLE 9

A solution may be prepared containing:-

| | |
|---|---|
| the compound of formula (I) | 15% w/v (weight/volume) |
| Dimethylsulphoxide | to 100% by volume |

by dissolving the pyrazole derivative in a portion of the dimethylsulphoxide and then adding more dimethylsulphoxide to the desired volume. This solution may be applied to domestic animals infested by arthropods, percutaneously as a pour-on application or, after sterilisation by filtration through a polytetrafluoroethylene membrane (0.22 micrometre pore size), by parenteral injection, at a rate of application of from 1.2 to 12 ml of solution per 100 kg of animal body weight.

### COMPOSITION EXAMPLE 10

A wettable powder may be formed from:-

| | |
|---|---|
| the compound of formula (I) | 50% w/w |
| Ethylan BCP (a nonylphenol/ethylene oxide condensate containing 9 moles of ethylene oxide per mol of phenol) | 5% w/w |
| Aerosil (silicon dioxide of microfine-particle size) | 5% w/w |
| Celite PF (synthetic magnesium silicate carrier) | 40% w/w |

by adsorbing the Ethylan BCP onto the Aerosil, mixing with the other ingredients and grinding the mixture in a hammer-mill to give a wettable powder, which may be diluted with water to a concentration of from 0.001% to 2% w/v of the pyrazole compound and applied to a locus of infestation by arthropods, for example dipterous larvae, or plant nematodes by spraying, or to domestic animals infested by, or at risk of infestation by, arthropods, helminths or protozoa, by spraying or dipping, or by oral administration in drinking water, to control the arthropods, helminths or protozoa.

### COMPOSITION EXAMPLE 11

A slow release bolus may be formed from granules containing a density agent, binder, slow-release agent and the compound of formula (I) at varying percentage compositions. By compressing the mixture a bolus with a specific gravity of 2 or more can be formed and may be administered orally to ruminant domestic animals for retention within the reticulo-rumen to give a continual slow release of pyrazole compound over an extended period of time to control infestation of the ruminant domestic animals by arthropods, helminths or protozoa.

### COMPOSITION EXAMPLE 12

A slow release composition may be prepared from:

| | |
|---|---|
| the compound of formula (I) | 0.5 to 25% w/w |
| polyvinylchloride base | to 100% w/w |

by blending the polyvinylchloride base with the pyrazole compound and a suitable plasticiser, e.g. dioctyl phthalate, and melt-extruding or hot-moulding the homogenous composition into suitable shapes, e.g. granules, pellets, brickettes or strips, suitable, for example, for addition to standing water or, in the case of strips, fabrication into collars or ear-tags for attachment to domestic animals, to control insect pests by slow release of the pyrazole compound.

The compound of general formula (I) can be prepared by the application or adaptation of known methods (ie. methods heretofore used or described in the chemical literature), generally pyrazole ring formation followed where necessary by changing substituents.

It is to be understood that in the description of the following processes that the sequences for the introduction of the various groups on the pyrazole ring may be performed in a different order and that suitable protecting groups may be required as will be apparent to those skilled in the art.

In the following description when symbols appearing in formulae are not specifically defined it is to be understood that they are "as hereinbefore defined" in accordance with the first definition of each symbol in this specification. Within the process definitions, unless otherwise stated, amino refers to the unsubstituted amino group.

The compound of formula (I) may be prepared by a process version "a" in which a compound of formula (II) wherein R⁸ represents a cyano group is reacted with a compound of formula R²CH₂CN, preferably a molar equivalent thereof, generally in the presence of an anhydrous inert organic solvent, e.g. ethanol, and a molar equivalent of a base, e.g. sodium ethoxide, and at a temperature from 0° to 50°C

According to a further process version "b", the compound of formula (I) may be prepared by oxidation of the sulphur atom of the corresponding compound of formula (I) in which R² is replaced by a group R⁵S as defined above; the oxidation may be effected by employing oxidants of the formula:-

R¹⁰-O-O-H (X)

wherein R¹⁰ represents hydrogen, or a trifluoroacetyl or preferably 3-chlorobenzoyl group in a solvent e.g. dichloromethane or chloroform or trifluoroacetic acid and at temperatures from 0°C to 60°C, or with a reagent such as potassium hydrogen persulphate or potassium salt of Caro's acid in a solvent e.g. methanol and water, and at a temperature from -30°C to 50°C.

According to a further process version "c" the compound of formula (I) may be prepared by the diazotisation of an intermediate corresponding to formula (I) in which R¹ is replaced by an amino group typically using sodium nitrite in a mineral acid, for example a mixture of concentrated sulphuric acid and acetic acid, at a temperature from 0° to 60°C, and by subsequent reaction with cuprous cyanide.

According to a further process version "d", the compound of formula (I) may be prepared by the reaction of a halide of the compound of formula (I) in which R¹ is replaced by a chlorine or bromine atom with a metal cyanide, preferably KCN typically under anhydrous conditions in an inert solvent, preferably sulpholane, and at a temperature from ambient to 150°C.

According to a further process version "e", the compound of formula (I) may also be prepared by the dehydration of a compound corresponding to formula (I) in which R¹ is replaced by the carbamoyl group. The compound corresponding to formula (I) in which R¹ is replaced a carbamoyl group may be prepared by the reaction of a compound corresponding to formula (I) in which R¹ is replaced a carboxy group with a chlorinating agent, preferably thionyl chloride at ambient to reflux temperature, followed by reaction of the intermediate acid chloride with ammonia to give an intermediate amide. The dehydration is generally effected by heating with a dehydrating agent e.g. phosphorus pentoxide or preferably phosphorus oxychloride at a temperature from 50°C to 250°C.

According to a further process version "f" the compound of formula (I) may be prepared by the conversion by known methods of a compound corresponding to formula (I) in which R³ represents a group -NR⁶R⁷ in which R⁶ represents an alkyl, alkenylalkyl or alkynylalkyl group containing up to 5 carbon atoms, formyl, or a group R¹¹C(O)- in which R¹¹ represents a straight- or branched-alkyl group containing 1 to 4 carbon atoms, R⁷ represents hydrogen or is as defined above for R⁶, or R⁶ and R⁷ together form a 5- or 6- membered cyclic imide into the compound of formula (I).

Intermediate compounds of the formula (II) in which the R⁸ group represents a cyano group may be prepared by diazotisation of the aniline R⁴NH₂ (in which R⁴ is as hereinbefore defined) generally with a solution of a molar equivalent of sodium nitrite in a mineral acid, e.g. a mixture of concentrated sulphuric acid and acetic acid, at a temperature from 0° to 60°C, and then reacting with a compound of formula CH₃COCH(Cl)CN [preparation described in J. Org. Chem 43 (2), 3822 (1978)] in the presence of an inert solvent, e.g. a mixture of water and ethanol, optionally buffered, e.g. with excess sodium acetate, and at a temperature from 0° to 50°C.

The intermediate compound of formula (I) in which R² is replaced by R⁵S may be prepared by reacting a compound of general formula (II) wherein R⁸ represents a cyano group with a compound of general formula R²CH₂CN, preferably a molar equivalent thereof, generally in the presence of an anhydrous inert organic solvent, e.g. ethanol, and a molar equivalent of a base, e.g. soidum ethoxide, and at a temperature from 0° to 50°C. Alternatively, the intermediate compound of formula (I) in which R² is replaced by R⁵S may be prepared by a process in which an intermediate corresponding to formula (I) in which R² is replaced by hydrogen is reacted with a compound of formula:-

R⁵SCl (III)

(in which R⁵ is as hereinbefore defined) in an inert organic solvent, preferably chloroform or dichloromethane, optionally in the presence of a base, preferably pyridine, and at temperatures from 0° to 50°C.

According to a further process the intermediate compound of formula (I) in which R² is replaced by R⁵S may also be prepared by the reaction of the corresponding 4-thiocyanatopyrazole with an organometallic reagent such as a compound of formula:-

R⁵-Mg-X¹ (VI)

in which R⁵ is as hereinbefore defined and X¹ represents a halogen atom in an inert solvent, such as diethyl ether or tetrahydrofuran, and at a temperature from -78°C to the reflux temperature of the reaction mixture or a compound of formula:-

R⁹-C≡C Li⁺ (IX)

in which R⁹-C≡C corresponds to R⁵ in (I), in an inert solvent, such as tetrahydrofuran or diethyl ether, at temperatures from -78°C to ambient.

Alternatively, according to a further process the intermediate compound of formula (I) in which R² is replaced by R⁵S may be prepared by reacting the corresponding compound of formula (I) in which R² is replaced by a thiocyanato group, with a base preferably sodium hydroxide, or a reducing agent preferably sodium borohydride, in the presence of a reagent of formula:

R⁵-X² (VII)

in which R⁵ is as hereinbefore defined and X² represents a halogen, preferably bromine or iodine, for example methyl iodide or propargyl bromide, or with a base preferably sodium hydroxide, in the presence of a reagent of formula:

F₂C=C(Z)Z' (VIIA)

in which Z represents a fluorine, chlorine or bromine atom and Z' is as hereinbefore defined for Z or represents a trifluoromethyl group in an inert organic or aqueous-organic solvent, such as methanol, ethanol or dioxan or mixtures of these solvents with water, the reaction being performed at a temperature from -40°C to the reflux temperature.

The intermediate corresponding to formula (I) in which R² is replaced by hydrogen may be prepared by diazotisation of the aniline R⁴NH₂ (in which R⁴ is as hereinbefore defined) generally with a solution of a molar equivalent of sodium nitrite in a mineral acid, e.g. a mixture of concentrated sulphuric acid and acetic acid, at a temperature from 0° to 65°C, and then reacting with a compound of formula:

NC-CH₂-CH(CO-R¹⁸)CN (XI)

in which R¹⁸ represents an alkoxy group containing from 1 to 6 carbon atoms, preferably an ethoxy group, or a hydrogen atom in the presence of an inert solvent, e.g. a mixture of water and ethanol, and optionally buffered, e.g. with sodium acetate, and at a temperature from 0 to 50°C. Subsequent mild hydrolysis with a base such as aqueous sodium hydroxide, sodium carbonate or ammonia may be necessary to effect the cyclisation.

The intermediate of formula (XI) used above, in which R¹⁸ represents the hydrogen atom, may be employed as alkali metal enolate salts which are converted into the aldehydes under the acidic conditions of the above coupling reaction.

The intermediate corresponding to formula (I) in which R² is replaced by hydrogen may also be prepared by decarboxylation of a compound corresponding to formula (I) in which R² is replaced by a carboxy group, generally performed by heating at a temperature from 100°C to 250°C optionally in the presence of an inert organic solvent, particularly N,N-dimethylaniline. Alternatively, the intermediate corresponding to formula (I) in which R² is replaced by a hydrogen atom may be prepared directly from esters corresponding to formula (I) in which R² is replaced by a group -COOR in which R represents a straight or branched chain alkyl group containing from 1 to 6 carbon atoms, by heating in an inert organic solvent preferably acetic acid at a temperature from 50°C to reflux, in the presence of a strong acid preferably hydrobromic acid. When the R¹ group is replaced by a chlorine or fluorine atom concomitant halogen exchange may also occur to give intermediates wherein R² and R³ are as defined and R¹ is replaced by a bromine atom.

The intermediate carboxy compound corresponding to formula (I) in which R² is replaced by a carboxy group may be prepared by hydrolysis of esters in which R² is replaced by a group -COOR as defined above, preferably with an alkali metal hydroxide in a solvent such as an aqueous alcohol at a temperature from 0°C to the reflux temperature of the reaction mixture.

Intermediate esters corresponding to formula (I) in which R² is replaced by a group -COOR as hereinbefore defined may be prepared in a similar manner to process version "a", described hereinbefore, from esters ROOCCH₂CN and intermediates of formula (II) in which R⁸ represents a cyano group.

Intermediate 4-thiocyanatopyrazoles corresponding to formula (I) in which R² is replaced by a thiocyanato group may be prepared by the reaction of a compound corresponding to formula (I) in which R² is replaced by hydrogen with a thiocyanating agent, such as alkali metal or ammonium salts of thiocyanic acid (e.g. NaSCN) and bromine, in an inert organic solvent, such as methanol, and at a temperature from 0°C to 100°C.

As a yet further feature of the invention the corresponding compound of formula (I) in which R² is replaced by R⁵S may be prepared by reductive alkylation of the disulphide of formula (VIII) employing a reducing agent preferably sodium dithionite or sodium borohydride, in the presence of a base, preferably sodium hydroxide or sodium carbonate, and of a halide of formula (VII), such as methyl iodide, in an inert organic or aqueous-organic solvent such as ethanol or a mixture of alcohol and water, at a temperature from ambient to reflux.

Intermediate disulphides of formula (VIII) may be prepared by the hydrolysis of thiocyanates in which R² is replaced by the thiocyanato group and R¹ represents a cyano group using hydrochloric acid in the presence of ethanol or by reduction with sodium borohydride in ethanol, both being at a temperature from ambient to reflux. Alternatively the thiocyanates may be converted into compounds of formula (VIII) by treatment with base, preferably aqueous sodium hydroxide and preferably under phase-transfer conditions with chloroform as co-solvent and in the presence of a phase transfer catalyst e.g. triethyl-benzylammonium chloride and at a temperature from ambient to 60°C.

The intermediate of formula (XIV) in which R¹⁹ represents an R² group or a hydrogen atom may be prepared by performing a Curtius rearrangement of the acid azide corresponding to formula (I) in which R¹ is replaced by CON₃ or in which R² is replaced by hydrogen and R¹ is replaced by CON₃ by heating in an inert organic solvent such as toluene at a temperature from 50°C to 150°C to give an isocyanate which is then reacted with for example tert-butanol to give a carbamate, which in turn is hydrolysed using dilute acid preferably hydrochloric acid in ethanol at a temperature from ambient to reflux.

Intermediate acid asides may be prepared by reaction of a carboxylic acid corresponding to formula (I) in which R¹ is replaced by a carboxy group with an azide transfer reagent such as diphenyl phosphoryl azide in the presence of a base, preferably triethylamine and in an inert solvent preferably N,N-dimethylformamide, and at a temperature from 0° to 60°C.

Intermediate carboxylic acids in which R¹ is replaced by a carboxylic acid group may be prepared by hydrolysis of the corresponding esters in which R¹ is replaced by an alkoxycarbonyl group e.g. ethoxycarbonyl, using a base such as sodium hydroxide and a solvent such as aqueous alcohol, and at a temperature from 0°C to the reflux temperature of the solvent.

Intermediate carboxylic esters in which R¹ represents an alkoxycarbonyl group and wherein R¹⁹ represents R² may be prepared by reaction of an intermediate (XV) in which R and R² are as hereinbefore defined and X⁶ is a leaving group, e.g. chlorine, with a phenylhydrazine (VI), in a similar manner to the process described above.

Intermediate carboxylic esters in which R¹ is replaced by an alkoxycarbonyl group as defined above, and R² is replaced by R¹⁹, may alternatively be prepared in a similar manner to process version "a" by the reaction of a compound (II) in which R⁸ is replaced by a -COOR group in which R is as hereinbefore defined, with a compound of formula R¹⁹CH₂CN in which R¹⁹ is as hereinbefore defined.

Intermediates corresponding to the formula (II) in which R⁸ is replaced by -COOR may be prepared from known compounds (e.g. CH₃COCH(Cl)COOR) in a similar manner to that described above for compounds of formula (II) in which R⁸ represents a cyano group.

Intermediate halides of formula (XV) in which X⁶ represents a chlorine atom and R and R² are as hereinbefore defined, may be prepared by the reaction of the sodium or potassium salts (XV) in which X⁶ is -O⁻Na⁺ or -O⁻K⁺ with a suitable chlorinating agent, preferably phosphorus oxychloride, optionally in the presence of an inert solvent, e.g. tetrahydrofuran, and at a temperature from 0°C to the reflux temperature of the solvent.

Intermediate salts (XV) in which X⁶ is -O⁻Na⁺ or -O⁻K⁺ may be prepared by methods described in the literature, wherein active methylene compounds R²CH₂CN are reacted with dialkyl oxalates, e.g. diethyl oxalate, in the presence of a metal alkoxide, e.g. sodium ethoxide, in an inert solvent, e.g. an alcohol such as ethanol, and at a temperature from 25°C to the reflux temperature of the solvent.

The intermediate compound of formula (I) in which R¹ is replaced by a chlorine atom, may be prepared by a process in which a compound of formula (IV) in which X and Y both represent chlorine atoms, is reacted with a phenylhydrazine of formula:-

R⁴NHNH₂ (V)

(in which R⁴ is as hereinbefore defined) or an acid addition salt thereof, e.g. the hydrochloride, in an inert solvent, preferably ether or tetrahydrofuran, and optionally in the presence of a base e.g. triethylamine or sodium acetate, and at a temperature from 0° to the reflux temperature of the solvent. When an acid addition salt of the compound of formula (V) is used, the reaction with the compound of formula (IV) is effected in the presence of an alkali metal, e.g. sodium or potassium, acetate, carbonate or bicarbonate.

Intermediate esters corresponding to formula (I) in which R² is replaced by a group -COOR as hereinbefore defined, R³ is the amino group and R¹ represents chlorine may be prepared by the reaction of a phenylhydrazine (V) with a compound of formula (XII) in which X, Y and R are as hereinbefore defined, in a similar manner to the procedure described above in which a compound of formula (IV) is reacted with a compound of formula (V).

Alternatively the intermediate corresponding to formula (I) in which R¹ is replaced by a chlorine atom, R² is replaced by a hydrogen atom, and R³ represents an amino group, may be prepared by reaction of the corresponding aldehydes in which R² is replaced by a formyl group with an acid, preferably aqueous hydrochloric acid, in a solvent preferably ethanol at a temperature from 50°C to the reflux temperature.

Intermediates corresponding to formula (I) in which R² is replaced by a formyl group may be prepared by reaction of nitriles in which R² is replaced by a cyano group with a suitable reducing agent, preferably diisobutyl aluminium hydride in an inert solvent, preferably tetrahydrofuran at a temperature from -78°C to ambient temperature.

Intermediates corresponding to formula (I) in which R² is replaced by a cyano group may be prepared by the reaction of a compound of formula (XIII) in which X and Y are as hereinbefore defined (i.e. dichlorodicyanoethylene or difluorodicyanoethylene), with a phenylhydrazine (V) in a similar manner to the process described above in which a compound of general formula (IV) is reacted with a compound of formula (V).

The intermediate compound of formula (I) in which R³ is replaced by a group -NHCH₂R¹⁶ in which R¹⁶ represents hydrogen may be prepared by a reaction of a compound of formula (I) in which R³ represents -N=C(OR¹⁷)R¹⁶ in which R¹⁷ represents a straight or branched-chain alkyl group containing from 1 to 4 carbon atoms with a reducing agent, preferably sodium borohydride. The reaction may be effected in an inert organic solvent, ethanol or methanol being preferred, at a temperature from 0°C to the reflux temperature of the reaction mixture.

According to a further feature of the present invention there are provided intermediates useful in the preparation of the compound of formula (I). These intermediates are represented by the following formulae: 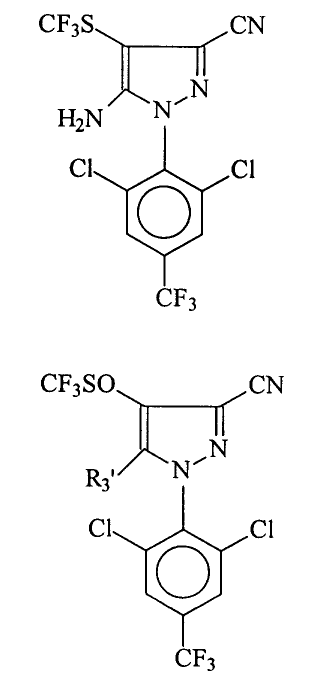 in which R₃' is a group -NR⁶R⁷ in which R⁶ represents an alkyl, alkenylalkyl or alkynylalkyl group containing up to 5 carbon atoms, formyl, or a group R¹¹C(O)- in which R¹¹ represents a straight- or branched-alkyl group containing 1 to 4 carbon atoms, R⁷ represents hydrogen or is as defined above for R⁶, or R⁶ and R⁷ together form a 5- or 6-membered cyclic imide.

The following Examples and Reference Examples illustrate the preparation of the compound of formula (I) according to the present invention: [Chromatography was effected on a silica column (May & Baker Ltd 40/60 flash silica) at a pressure of 6.8Nm⁻², unless otherwise stated.]

### EXAMPLE 1

### The compound of general formula (I)

A stirred solution of 5-amino-3-cyano-1-(2,6-dichloro-4-trifluoromethylphenyl)-4-trifluoromethylthiopyrazole (10.0g) in dichloromethane (100ml) was treated with m-chloroperbenzoic acid (4.5g). After stirring overnight additional m-chloroperbenzoic acid (1.6g) was added in 2 portions, and left for 2 days. The reaction product was diluted with ethyl acetate (30ml) and then washed in turn with sodium sulphite solution (50ml), sodium carbonate solution (50ml) and with water (50ml). After drying over magnesium sulphate, this was filtered and evaporated in vacuo. Purification by chromatography on silica eluting with dichloromethane gave 5-amino-3-cyano-1-(2,6-dichloro-4-trifluoromethylphenyl)-4-trifluoromethylsulphinylpyrazole as a white solid (6.0g), m.p. 200.5-201°C.

### REFERENCE EXAMPLE 1

A solution of 5-amino-3-cyano-1-(2,6-dichloro-4-trifluoromethylphenyl)pyrazole (20.0g) in dichloromethane (100ml) was stirred magnetically and treated dropwise with a solution of trifluoromethylsulphenyl chloride (10.8g) in dichloromethane (50ml) during 1 hour. The solution was stirred overnight at room temperature, then washed with water (100ml), dried over anhydrous magnesium sulphate, filtered, and evaporated in vacuo to give a solid (26.3g). This solid was recrystallised from toluene/hexane to give 5-amino-3-cyano-1-(2,6-dichloro-4-trifluoromethylphenyl)-4-trifluoromethylthiopyrazole as fawn crystals (24.2g) m.p. 169-171°C.

### REFERENCE EXAMPLE 2

5-Amino-3-cyano-1-(2,6-dichloro-4-trifluoromethylphenyl)pyrazole was prepared as follows:
A suspension of nitrosyl sulphuric acid prepared from sodium nitrite (7.0g) and concentrated sulphuric acid (27.5ml) was diluted with acetic acid (25ml), cooled to 25°C, and stirred mechanically. To this was added a solution of 2,6-dichloro-4-trifluoromethylaniline (21.2g) in acetic acid (50ml) dropwise over 15 minutes at 25-32°C. This mixture was heated to 55°C for 20 minutes and poured onto a stirred solution of ethyl-2,3-dicyanopropionate (14.0g) in acetic acid (60ml) and water (125ml) at 10-20°C. After 15 minutes, water (200ml) was added, and the oily layer separated. The aqueous solution was then extracted with dichloromethane (3x 70ml) and the extracts combined with the oil and washed with ammonia solution (to pH9). The organic phase was then stirred with ammonia (20ml) for 2 hours, and the dichloromethane layer then separated. This was washed with water (1x 100ml), 1N hydrochloric acid (1x 100ml), dried over anhydrous magnesium sulphate, filtered, and evaporated in vacuo to give an oily solid. Crystallisation from toluene/hexane gave the title compound as brown crystals (20.9g), m.p. 140-142°C.

### REFERENCE EXAMPLE 3

To a stirred solution of 5-amino-3-cyano-1-(2,6-dichloro-4-trifluoromethy]phenyl)pyrazole (2.23 g) and pyridine (0.55 g) in chloroform (50 ml) was added dropwise at 0°C, a solution of trifluoromethylsulphenyl chloride (1.24 g) in chloroform (15 ml) during 20 minutes. The mixture was stirred at 0°C for 3 hours, and the solvent evaporated in vacuo to give a yellow solid (3.1 g). This was purified by chromatography on silica (Merck, 230-400 mesh, 0.7 kgcm⁻²) eluting with dichloromethane and petroleum ether b. 40-60° (3:1) to give 5-amino-1-(2,6-dichloro-4-trifluoromethylphenyl)-3-cyano-4-trifluoromethylthiopyrazole in the form of a white solid (2.33 g), m.p. 169.5-170.5°C.

### EXAMPLE 2

### The Compound of formula (I)

By proceeding in a similar manner to that described below but replacing 5-amino-1-(2,6-dichloro-4-trifluoromethylphenyl)-4-methylthio-3-trifluoromethylpyrazole by the following phenylpyrazole, there was obtained:-
5-Amino-1-(2,6-dichloro-4-trifluoromethylphenyl)-3-cyano-4-trifluoromethylsulphinylpyrazole in the form of white solid, m.p. 203-203.5°C from 5-amino-1-(2,6-dichloro-4-trifluoromethylphenyl)-3-cyano-4-trifluoromethylthiopyrazole.

A stirred solution of 5-amino-1-(2,6-dichloro-4-trifluoromethylphenyl)-4-methylthio-3-trifluoromethylpyrazole (1.0 g) in chloroform (40 ml) was treated with m-chloroperbenzoic acid (0.42 g), portionwise at room temperature. After stirring for 6 hours, the solution was diluted with dichloromethane and washed in turn with sodium sulphite solution, sodium hydroxide solution, and water. The solution was dried over anhydrous magnesium sulphate, and evaporated in vacuo to give a yellow oil. Purification by chromatography on silica (Merck, 230-400 mesh, 0.7 kg cm⁻²) eluting with dichloromethane-ethylacetate (4:1) gave 5-amino-1-(2,6-dichloro-4-trifluoromethylphenyl)-4-methylsulphinyl-3-trifluoromethylpyrazole in the form of a white solid, m.p. 142-145°C with decomposition.

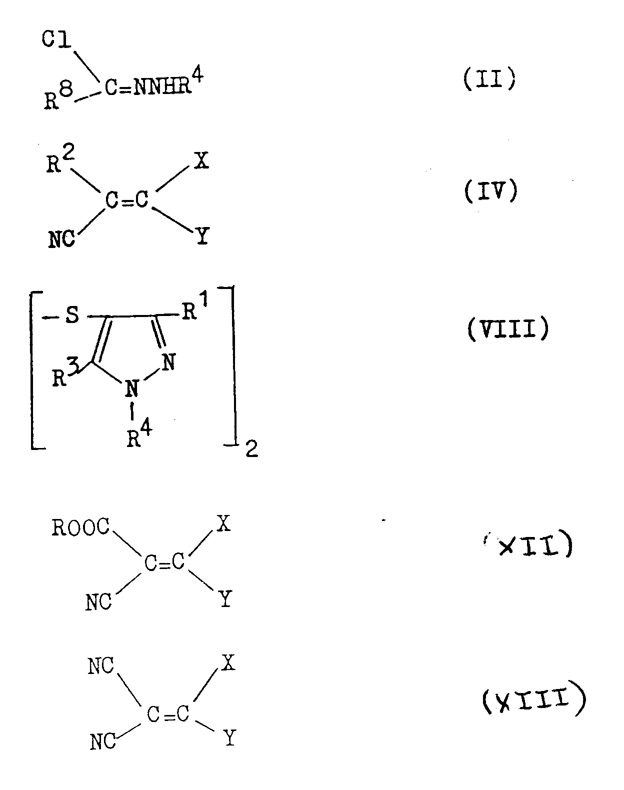

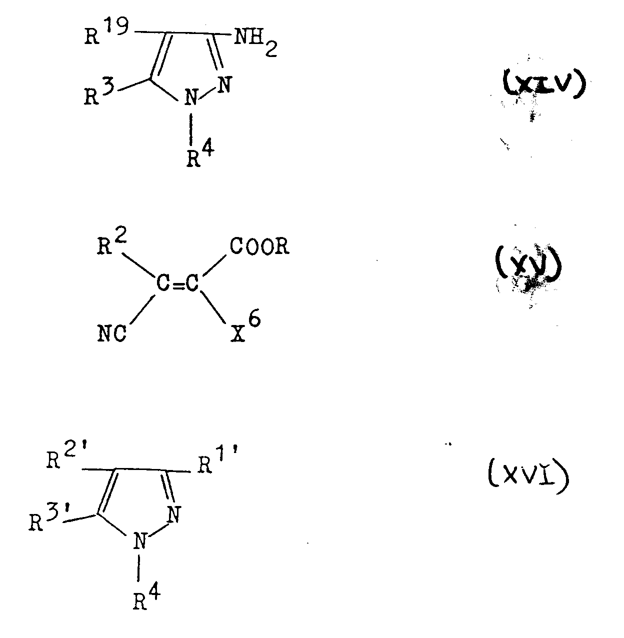

## Claims

1. An N-phenylpyrazole derivative of the formula:

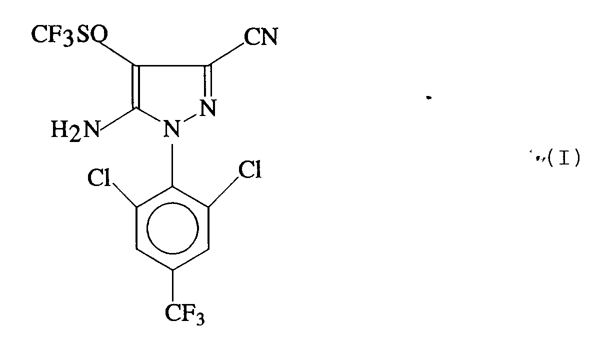

2. A process for the preparation of the compound of formula (I) as claimed in claim 1 which comprises:
(a) the reaction of a compound of formula: 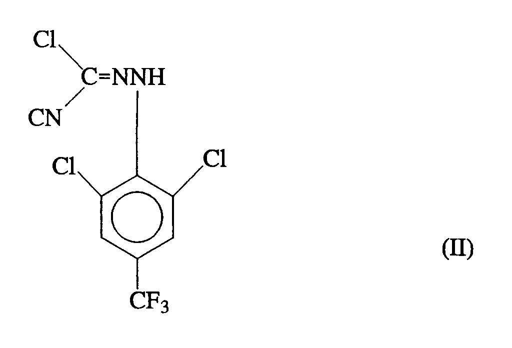 with a compound of the formula CF₃SOCH₂CN;
(b) the oxidation of the sulphur atom in a compound of formula (I) as claimed in claim 1 in which the trifluoromethylsulphinyl group is replaced by a trifluoromethylthio group;
(c) the diazotisation of a compound corresponding to formula (I) as claimed in claim 1 in which the cyano group is replaced by an amino group, and conversion of the diazotised amino group into a cyano group by known methods;
(d) the reaction of a compound corresponding to formula (I) as claimed in claim 1 in which the cyano group is replaced by a chlorine or bromine atom with a metal cyanide to convert the chlorine or bromine atom to a cyano group;
(e) the dehydration of a compound corresponding to formula (I) as claimed in claim 1 in which the cyano group is replaced by a carbamoyl group; or
(f) the conversion by known methods of a compound corresponding to formula (I) as claimed in claim 1 in which the amino group is replaced by a group -NR⁶R⁷ in which R⁶ represents an alkyl, alkenylalkyl or alkynylalkyl group containing up to 5 carbon atoms, formyl, or a group R¹¹C(O)- in which R¹¹ represents a straight- or branched-alkyl group containing 1 to 4 carbon atoms, R⁷ represents hydrogen or is as defined above for R⁶, or R⁶ and R⁷ together form a 5- or 6- membered cyclic imide into the compound of formula (I).

3. Anarthropodicidal, plant nematocidal, anthelmintic or anti-protozoal composition which comprises the compound of formula (I) as claimed in claim 1, in association with one or more compatible diluents or carriers.

4. A method for the control of arthropod, plant nematode, helminth or protozoan pests at a locus which comprises treatment of the locus with an effective amount of the compound claimed in claim 1, with the exclusion of a method for the treatment of the human or animal body carried out by a medical or veterinary practitioner as therapy.

5. A compound of formula (I) as claimed in claim 1 for use in the manufacture of a medicament for the treatment of an arthropod, helminth or protozoal infection.

6. A compound of formula:

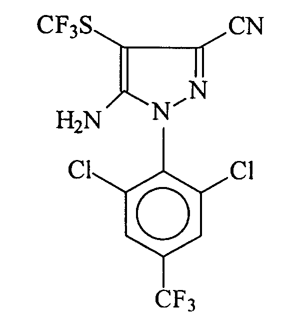

7. A compound of formula: 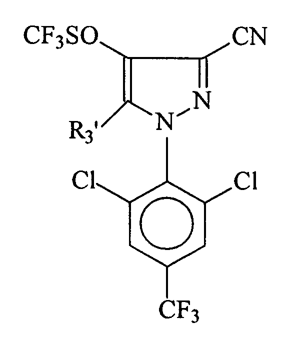 in which R₃' is a group -NR⁶R⁷ in which R⁶ represents an alkyl, alkenylalkyl or alkynylalkyl group containing up to 5 carbon atoms, formyl, or a group R¹¹C(O)- in which R¹¹ represents a straight- or branched-alkyl group containing 1 to 4 carbon atoms, R⁷ represents hydrogen or is as defined above for R⁶, or R⁶ and R⁷ together form a 5- or 6- membered cyclic imide.

## Patentansprüche

1. N-Phenylpyrazolderivat-der-Formel:

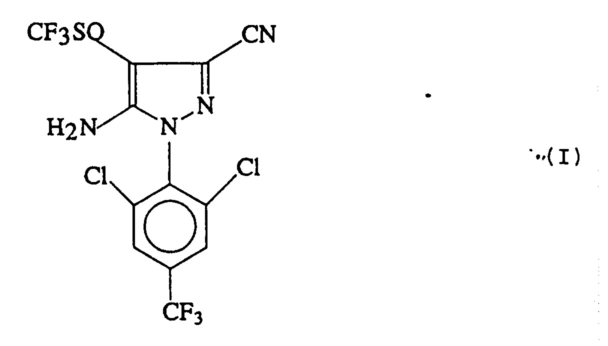

2. Verfahren zur Herstellung der Verbindung der Formel (I) gemäß Anspruch 1, umfassend:
(a) die Reaktion einer Verbindung der Formel 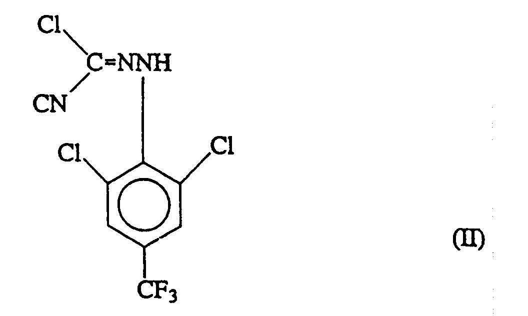 mit einer Verbindung der Formel CF₃SOCH₂CN;
(b) die Oxidation des Schwefelatoms in einer Verbindung der Formel (I) gemäß Anspruch 1, wobei die Trifluormethylsulphinylgruppe durch eine Trifluormethylthiogruppe ersetzt wird;
(c) die Diazotierung einer Verbindung entsprechend der Formel (I) gemäß Anspruch 1, wobei die Cyanogruppe durch eine Aminogruppe ersetzt wird, und die Umwandlung der diazotierten Aminogruppe in eine Cyanogruppe durch bekannte Methoden;
(d) die Reaktion einer Verbindung entsprechend der Formel (I) gemäß Anspruch 1, wobei die Cyanogruppe durch ein Chlor- oder Bromatom mit einem Metallcyanid ersetzt wird, um das Chlor- oder Bromatom zu einer Cyanogruppe umzuwandeln;
(e) die Dehydratisierung einer Verbindung entsprechend der Formel (I) gemäß Anspruch 1, wobei die Cyanogruppe durch eine Carbamoylgruppe ersetzt wird; oder
(f) die Umwandlung durch bekannte Methoden einer Verbindung entsprechend der Formel (I) gemäß Anspruch 1, wobei die Aminogruppe durch eine -NR⁶R⁷-Gruppe, worin R⁶ für eine Alkyl-, Alkenylalkyl- oder Alkynylalkylgruppe mit bis zu 5 Kohlenstoffatomen steht, Formyl oder eine R¹¹C(O)-Gruppe, worin R¹¹ für eine geradkettige oder verzweigtkettige Alkylgruppe mit 1 bis 4 Kohlenstoffatomen steht, ersetzt wird, R⁷ für Wasserstoff steht oder wie obenstehend für R⁶ definiert ist, oder R⁶ und R⁷ zusammen ein 5- oder 6-gliedriges cyclisches Imid bilden, zu der Verbindung der Formel (I).

3. Arthropodizide, pflanzennematozide, anthelmintische oder antiprotozoale Zusammensetzung, welche die Verbindung der Formel (I) gemäß Anspruch 1 umfaßt, in Verbindung mit einem oder mehreren kompatiblen Verdünnungsmitteln oder Trägern.

4. Verfahren zur Bekämpfung arthropodizider, pflanzennematozider, helminthischer oder protozoaner Schädlinge an einem Ort bzw. Locus, welches die Behandlung des Ortes mit einer wirksamen Menge der Verbindung gemäß Anspruch 1 umfaßt, unter Ausschluß eines Verfahrens zur Behandlung des menschlichen Körpers oder des Körpers von Tieren, das durch einen Mediziner oder Tiermediziner als Therapie durchgeführt wird.

5. Verbindung der Formel (I) gemäß Anspruch 1 zur Verwendung bei der Herstellung eines Medikaments zur Behandlung einer arthropodiziden, helminthischen oder protozoalen Infektion.

6. Verbindung der Formel:

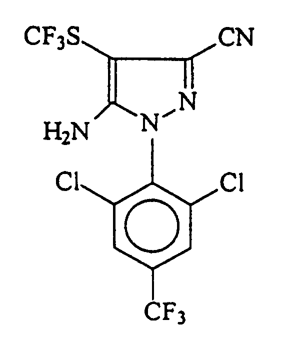

7. Verbindung der Formel: 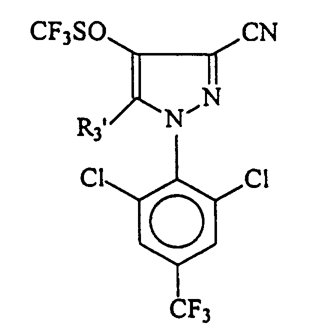 worin R₃' eine -NR⁶R⁷-Gruppe ist, in welcher R⁶ für eine Alkyl-, Alkenylalkyl- oder Alkynylalkylgruppe mit bis zu 5 Kohlenstoffatomen, Formyl oder eine R¹¹C(O)-Gruppe steht, in welcher R¹¹ für eine geradkettige oder verzweigtkettige Alkylgruppe mit 1 bis 4 Kohlenstoffatomen steht, R⁷ für Wasserstoff steht oder wie obenstehend für R⁶ definiert ist, oder R⁶ und R⁷ zusammen ein 5- oder 6-gliedriges cyclisches Imid bilden.

## Revendications

1. Dérivé de N-phénylpyrazole de formule :

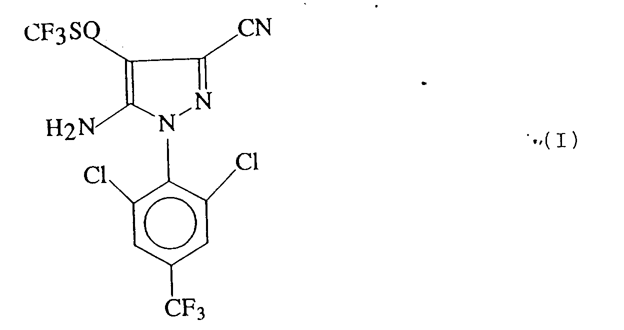

2. Procédé pour la préparation du composé de formule (I) suivant la revendication 1, qui comprend :
(a) la réaction d'un composé de formule : 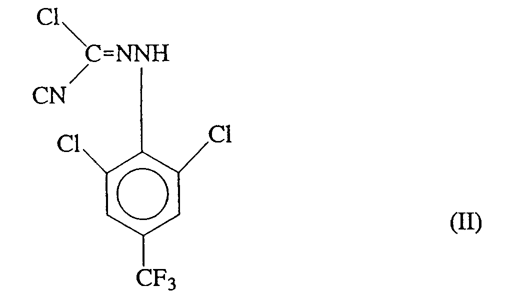 avec un composé de formule CF₃SOCH₂CN ;
(b) l'oxydation de l'atome de soufre dans un composé de formule (I) suivant la revendication 1 dans lequel le groupe trifluorométhylsulfinyle est remplacé par un groupe trifluorométhylthio ;
(c) la diazotation d'un composé répondant à la formule (I) suivant la revendication 1, dans laquelle le groupe cyano est remplacé par un groupe amino, et la conversion du groupe amino diazoté en un groupe cyano par des procédés connus ;
(d) la réaction d'un composé répondant à la formule (I) suivant la revendication 1, dans laquelle le groupe cyano est remplacé par un atome de chlore ou de brome avec un cyanure métallique pour remplacer l'atome de chlore ou de brome par un groupe cyano ;
(e) la déshydratation d'un composé répondant à la formule (I) suivant la revendication 1 dans laquelle le groupe cyano est remplacé par un groupe carbamoyle ; ou
(f) la conversion par des procédés connus d'un composé répondant à la formule (I) suivant la revendication 1, dans laquelle le groupe amino est remplacé par un groupe -NR⁶R⁷ dans lequel R⁶ représente un groupe alkyle, alcénylalkyle ou alcynylalkyle contenant jusqu'à 5 atomes de carbone, un groupe formyle ou un groupe R¹¹C(O)- dans lequel R¹¹ représente un groupe alkyle droit ou ramifié contenant 1 à 4 atomes de carbone, R⁷ représente l'hydrogène ou répond à la définition précitée pour R⁶, ou bien R⁶ et R⁷, conjointement, forment un imide cyclique penta- ou hexagonal, en le composé de formule (I).

3. Composition pour la destruction d'arthropodes, de nématodes des végétaux, d'helminthes ou de protozoaires, qui comprend le composé de formule (I) suivant la revendication 1, en association avec un ou plusieurs diluants ou supports compatibles.

4. Méthode de lutte contre des arthropodes, des nématodes des végétaux, des helminthes ou des protozoaires parasites dans un milieu, qui comprend le traitement de ce milieu avec une quantité efficace du composé suivant la revendication 1, à l'exclusion d'une méthode pour le traitement de l'organisme de l'homme ou d'un animal mise en oeuvre par un médecin ou vétérinaire à titre thérapeutique.

5. Composé de formule (I) suivant la revendication 1, destiné à être utilisé dans la production d'un médicament pour le traitement d'une affection par des arthropodes, des helminthes ou des protozoaires.

6. Composé de formule :

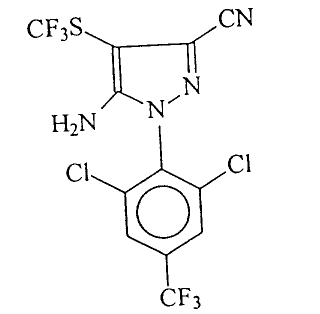

7. Composé de formule : 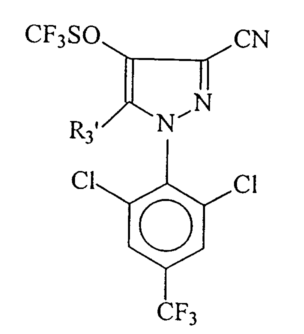 dans laquelle R₃' représente un groupe -NR⁶R⁷ dans lequel R⁶ représente un groupe alkyle, alcénylalkyle ou alcynylalkyle contenant jusqu'à 5 atomes de carbone, un groupe formyle ou un groupe R¹¹C(O)- dans lequel R¹¹ représente un groupe alkyle droit ou ramifié contenant 1 à 4 atomes de carbone, R⁷ représente l'hydrogène ou répond à la définition précitée pour R⁶, ou bien R⁶ et R⁷, conjointement, forment un imide cyclique penta- ou hexagonal.
